# EUROPEAN PATENT APPLICATION

(11) **EP 3 351 940 A1**
(43) Date of publication of application: **25.07.2018**
(21) Application number: 17152913.4
(22) Date of filing: 24.01.2017
(51) Int. Cl.: G01N 35/04, B65G 54/02, B65G 1/00

(54) **LABORATORY WITH SAMPLE DISTRIBUTION IN UNDERFLOOR CHAMBER**

(71) Applicant: Roche Diagnostics GmbH, 68305 Mannheim (DE); F. Hoffmann-La Roche AG, 4070 Basel (CH)
(72) Inventor: Van Mierlo, Hans, 5141 HC Waalwijk (NL)
(74) Representative: Patentanwälte Ruff, Wilhelm, Beier, Dauster & Partner mbB

(57) **Abstract**

The invention relates to a laboratory with a laboratory sample distribution system being at least partially arranged in an underfloor chamber.

## Description

### Applicable field and prior art

The invention relates to a laboratory comprising a laboratory room. Such a laboratory may typically comprise a number of analytical stations and a laboratory sample distribution system being adapted to distribute sample container carriers or sample containers between the stations.

Typical laboratory sample distribution systems allow for high throughput and for reliable operation. An exemplary laboratory sample distribution system is shown in document WO 2013/064656 A1.

It has been found that in typical laboratories problems may arise due to complex laboratory layouts. For example, the laboratory sample distribution system should be able to distribute samples or sample containers between the stations as flexibly as possible, which requires using a substantial part of the laboratory space for a sufficiently large transport plane. However, this requirement deteriorates accessibility of the stations by operators and makes it difficult to implement emergency escape routes.

### Object and solution

It is thus an object of the invention to provide for a laboratory that is optimized regarding the problems mentioned above.

This object is solved by a laboratory according to claim 1.

The invention relates to a laboratory. The laboratory comprises a laboratory room or laboratory space and an underfloor chamber located below or under the laboratory room or laboratory space.

The laboratory comprises a number (e.g. 1 to 50) of pre-analytical, analytical and/or post-analytical stations.

The laboratory comprises a number (e.g. 1 to 10.000) of sample container carriers being adapted to carry the sample containers comprising samples to be processed by the stations. The laboratory comprises a laboratory sample distribution system being adapted to distribute the sample container carriers between the stations, wherein the laboratory sample distribution system comprises:
- a transport plane,
- drive means being adapted to move the sample container carriers on the transport plane, and
- a control device being configured to control the movement of the sample container carriers on top of the transport plane by driving the drive means such that the sample container carriers move along corresponding transport paths,
- wherein the laboratory sample distribution system is at least partially arranged inside the underfloor chamber.

By means of the inventive laboratory, at least a part of the laboratory sample distribution system can be arranged inside the underfloor chamber, thus leaving free space in the laboratory room that can be used, for example, to provide for emergency exit routes or to allow accessibility of the stations by operators.

Further, distribution of consumables can be simplified as they can, at least partially, be provided using the underfloor chamber.

The underfloor chamber can in principle be provided by any kind of hollow interior below the laboratory room. For example, the underfloor chamber can be provided under the whole laboratory room, or under a part of the laboratory room.

The stations may be arranged adjacent to the laboratory sample distribution system.

Pre-analytical stations may be adapted to perform any kind of pre-processing of samples, sample containers and/or sample container carriers.

Analytical stations may be adapted to use a sample or part of the sample and a reagent to generate a measuring signal, the measuring signal indicating if and in which concentration, if any, an analyte is existing.

Post-analytical stations may be adapted to perform any kind of post-processing of samples, sample containers and/or sample container carriers.

The pre-analytical, analytical and/or post-analytical stations may comprise at least one of a decapping station, a recapping station, an aliquot station, a centrifugation station, an archiving station, a pipetting station, a sorting station, a tube type identification station, a sample quality determining station, an add-on buffer station, a liquid level detection station, and a sealing/desealing station. The sample container carriers can be regarded as part of the laboratory sample distribution system. However, the sample container carriers can also be regarded as separate from the sample distribution system.

Sample containers are typically designed as tubes made of glass or transparent plastic and typically have an opening at an upper end. The sample containers can be used to contain, store and transport samples such as blood samples or chemical samples.

The transport plane can also be denoted as transport surface. The transport plane supports the sample container carriers, what can also be denoted as carrying the sample container carriers.

The control device is typically a microprocessor, a microcontroller, a field programmable gate array, a standard computer, or a similar device. In a typical embodiment, the control device comprises processor means and storage means, wherein program code is stored in the storage means in order to control the behavior of the processor means when the storage code is executed on the processor means.

The sample container carriers are typically adapted to move in two dimensions on the transport plane.

According to an embodiment, the transport plane is arranged inside the underfloor chamber. The transport plane can be arranged completely inside the underfloor chamber. This allows for solely using the underfloor chamber for transporting the sample container carriers.

According to an embodiment, the transport plane comprises a number (e.g. 1 to 100) of sections being arranged above or outside the underfloor chamber. The transport plane may comprise a number (e.g. 1 to 100) of sections being arranged inside the underfloor chamber. This allows for a suitable distribution of transport plane sections between the laboratory room and the underfloor chamber.

According to an embodiment, the laboratory sample distribution system further comprises a vertical transport means, wherein the vertical transport means is configured to transport sample container carriers or sample containers between the transport plane and a location having a vertical level being different from a vertical level of the transport plane. Such a vertical transport means allows for a distribution of a sample container carrier from a transport plane to a station even if the station is located at another level than the transport plane. For example, the station can be placed inside the laboratory room, and the transport plane or the relevant section of the transport plane can be placed inside the underfloor chamber, or vice versa.

Vertical transport means can also be used to transport sample containers or sample container carriers between sections of the transport plane having different vertical levels.

According to an embodiment, the underfloor chamber is covered by a number (e.g. 1 to 1000) of releasable floor tiles. This allows for easy access to the underfloor chamber by simply removing the floor tiles or a part of the floor tiles.

According to an embodiment, the floor tiles are embodied to provide a walk-in ground floor. This allows for easy access of operators to locations in the laboratory room, wherein the tiles covering the underfloor chamber can be used as a walking floor. Also a higher load resistance is possible, if needed.

According to an embodiment, some or all of the floor tiles are optically transparent. This allows for viewing operation of the parts of the laboratory sample distribution system being arranged below the laboratory room, which would otherwise not be viewable during normal operation. For example, errors, if any, can be detected.

According to an embodiment, the underfloor chamber comprises a number (e.g. 1 to 100) of supply means, preferably electrical or fluid supply means. This allows for at least partially or wholly providing supply means using the underfloor chamber, which can also save limited space in the laboratory room.

According to an embodiment, the laboratory comprises a plurality of underfloor chambers, wherein the laboratory sample distribution system is at least partially arranged inside the underfloor chambers. This allows distribution of the laboratory sample distribution system over a plurality of underfloor chambers.

According to an embodiment, the laboratory comprises sealant means being adapted to seal the underfloor chamber liquid-tight. This allows preventing liquids flowing into the underfloor chamber, those liquids possibly preventing safe operation or contaminating samples to be analyzed. For example, such sealant means can be arranged around the floor tiles.

According to an embodiment, the laboratory sample distribution system comprises underfloor storage means to temporarily store a number (e.g. 1 to 10.000) of sample container carriers or sample containers, wherein the underfloor storage means are arranged inside the underfloor chamber. This allows for at least temporarily storing sample container carriers using the underfloor chamber, which can also save space in the laboratory room that would otherwise be needed for storage means in the laboratory room.

According to an embodiment, the drive means are embodied as electro-magnetic actuators, and each sample container carrier comprises a magnetically active device, for example a permanent magnet. This allows for efficient and reliable operation.

According to a further embodiment, the drive means are formed as wheels driven by electric motors being located in the sample container carriers and being controllable by the control device.

According to an embodiment, the transport plane is arranged solely inside the underfloor chamber, at least one station of the stations is arranged in the underfloor chamber, and at least one station of the stations is arranged above the underfloor chamber. This allows for a suitable distribution of the stations between the underfloor chamber and the laboratory room.

### Short description of the drawing

The invention will be described in detail with respect to the drawing schematically depicting an embodiment of the invention. In detail:

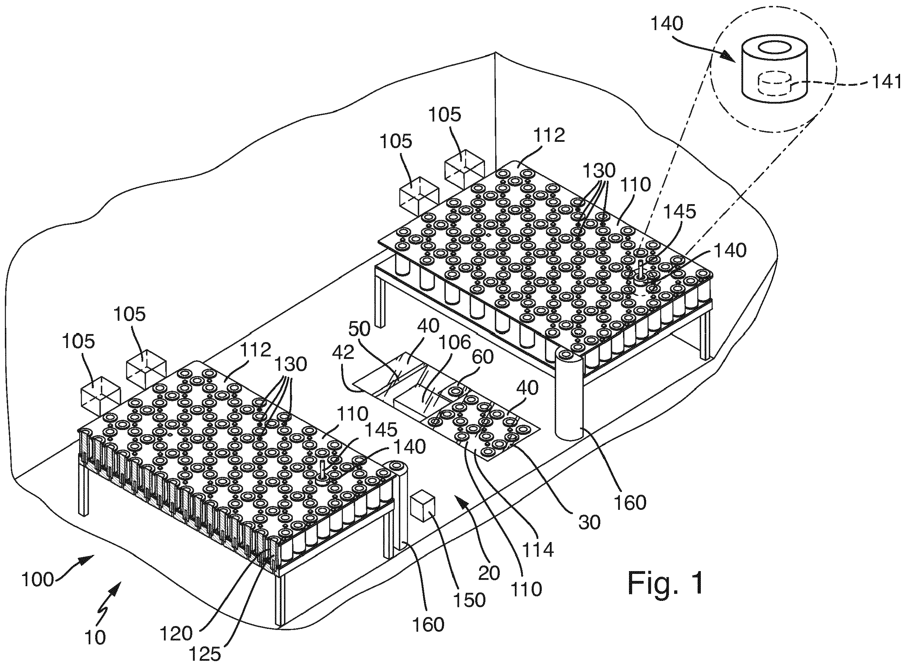

Fig. 1 shows a laboratory according to the invention.

### Detailed description of the embodiment

Fig. 1 shows a laboratory 10. The laboratory 10 comprises a laboratory room 20 and an underfloor chamber 30 being arranged below or under the laboratory room 20. The underfloor chamber 30 is accessible by two removable floor tiles 40.

The floor tiles 40 are optically transparent so that it is possible to see into the underfloor chamber 30. The floor tiles 40 are dimensioned to withstand the weight of a person standing on it, so that the floor tiles 40 provide for a walk-in floor.

The floor tiles 40 are surrounded by a sealant means 42 being liquid-tight so as to prevent liquids or other contaminants from entering the underfloor chamber 30.

The laboratory 10 comprising a laboratory sample distribution system 100 and a number of pre-analytical, analytical and/or post-analytical stations 105, 106 arranged adjacent to the laboratory sample distribution system 100. Self-evidently, more than the altogether five stations 105, 106 schematically depicted in fig. 1 may be comprised in the laboratory sample distribution system 100. In the shown example, four stations 105 are arranged in the laboratory room 20, and one station 106 is arranged in the underfloor chamber 30.

The laboratory sample distribution system 100 comprises a transport plane 110 below which a plurality of electro-magnetic actuators in the form of electromagnets 120 are positioned. The electromagnets 120 are implemented as solenoids having solid ferromagnetic cores 125.

The transport plane 110 has sections 112 being provided in the laboratory room 20 and sections 114 being provided in the underfloor chamber 30. Thus, the transport plane 110 is distributed between the laboratory room 20 and the underfloor chamber 30.

Sample container carriers 140 respectively comprising a magnetically active device 141 in form of a permanent magnet are positioned on the transport plane 110 and can be moved by means of the electromagnets 120. While it should be understood that a plurality of sample container carriers 140 can be positioned on the transport plane 110, due to simplicity only one respective sample container carrier 140 on each of the two sections 112 of the transport plane 110 in the laboratory room 20 is depicted in fig. 1. Each sample container carrier 140 holds a respective sample container 145, in which a sample to be analyzed can be contained.

The laboratory sample distribution system 100 is adapted to transport the sample container carriers 140 and/or the sample containers 145 between the laboratory stations 105, 106. The laboratory stations 105, 106 are positioned adjacent to the transport plane 110 such that a sample container carrier 140 can be used to transport a sample contained in the sample container 145 to a respective laboratory station 105, 106.

A plurality of Hall-sensors 130 is arranged such that positions of respective sample container carriers 140 on the transport plane 110 can be detected.

The laboratory sample distribution system 100 further comprises a control device 150. The control device 150 is configured to control movement of the sample container carriers 140 on the transport plane 110 by driving the electromagnets 120 such that the sample container carriers 140 independently and simultaneously move along corresponding transport paths.

The laboratory sample distribution system 100 further comprises two vertical transport means 160. As shown, one respective vertical transport means 160 is arranged besides each of the two sections 112 of the transport plane 110 in the laboratory room 20.

The vertical transport means 160 are embodied as vertically moving planes on which a sample container carrier 140 can be positioned and transported to a different level. In the present case, each vertical transport means 160 is configured to transport a sample container carrier 140 between a section 112 in the laboratory room and the section 114 in the underfloor chamber 30. Thus, sample container carriers 140 and sample containers 145 can be moved on the entire transport plane 110. For example, a sample container carrier 140 carrying a sample container 145 can be moved from one of the two sections 112 in the laboratory room 20 to the section 114 in the underfloor chamber 30 and can then be moved further to the other section 112 in the laboratory room 20.

For holding the sample container carrier 140 to be transported, each vertical transport means 160 comprises an electro-magnetic actuator similarly to the electro-magnetic actuators 120.

Given the functionality just described, sample container carriers 140 can be distributed freely over the entire transport plane 110 using sections 112, 114 both in the laboratory room 20 and in the underfloor chamber 30. However, in contrast to systems known in the prior art, an empty space is left in the laboratory room 20 between the two sections 112 of the transport plane 110. This can, for example, be used for maintenance purposes or as an emergency escape route.

The shown station 106 being positioned in the underfloor chamber 30 can also be used similarly to the stations 105 being positioned in the laboratory room 20.

An underfloor storage means 60 is arranged inside the underfloor chamber 30 to temporarily store sample container carriers 140. The underfloor storage means 60 is embodied as a horizontal extension of the section 114.

In addition, supply means 50 in form of a fluid pipe are arranged inside the underfloor chamber 30. The fluid pipe can be used to supply fluids to the stations 105, 106. Usage of the underfloor chamber 30 for this purpose has the advantage that these supply means 50 do not occupy space in the laboratory room 20. Similarly, the underfloor chamber 30 can be used to provide other consumables, electricity or data communications means, etc.

## Claims

1. Laboratory (10), comprising
- a laboratory room (20),
- an underfloor chamber (30) located below the laboratory room (20),
- a number of pre-analytical, analytical and/or post-analytical stations (105, 106),
- a number of sample container carriers (140) being adapted to carry sample containers (145) comprising samples to be processed by the stations (105, 106), and
- a laboratory sample distribution system (100) being adapted to distribute the sample container carriers (140) between the stations (105, 106), wherein the laboratory sample distribution system (100) comprises:
- a transport plane (110),
- drive means (120) being adapted to move the sample container carriers (140) on the transport plane (110), and
- a control device (150) being configured to control the movement of the sample container carriers (140) on top of the transport plane (110) by driving the drive means (120) such that the sample container carriers (140) move along corresponding transport paths,
- wherein the laboratory sample distribution system (100) is at least partially arranged inside the underfloor chamber (30).

2. Laboratory (10) according to claim 1,
**characterized in that**
- the transport plane (110) is arranged inside the underfloor chamber (30).

3. Laboratory (10) according to claim 1,
**characterized in that**
- the transport plane (110) comprises a number of sections (112) being arranged above the underfloor chamber (30).

4. Laboratory (10) according to one of the preceding claims,
**characterized in that**
- the laboratory sample distribution system (100) further comprises a vertical transport means (160), wherein the vertical transport means (160) is configured to transport sample container carriers (140) or sample containers (145) between the transport plane (110) and a location having a vertical level being different from a vertical level of the transport plane (110).

5. Laboratory (10) according to one of the preceding claims,
**characterized in that**
- the underfloor chamber (30) is covered by a number of releasable floor tiles (40).

6. Laboratory (10) according to claim 5,
**characterized in that**
- the floor tiles (40) are embodied to provide a walk-in ground floor.

7. Laboratory (10) according to claim 5 or 6,
**characterized in that**
- some or all of the floor tiles (40) are optically transparent.

8. Laboratory (10) according to one of the preceding claims,
**characterized in that**
- the underfloor chamber (30) comprises a number of supply means (50), preferably electrical or fluid supply means.

9. Laboratory (10) according to one of the preceding claims,
**characterized in that**
- the laboratory (10) comprises a plurality of underfloor chambers (30), wherein the laboratory sample distribution system (100) is at least partially arranged inside the underfloor chambers (30).

10. Laboratory (10) according to one of the preceding claims,
**characterized in that**
- the laboratory (10) comprises sealant means (42) being adapted to seal the underfloor chamber (30) liquid-tight.

11. Laboratory (10) according to one of the preceding claims,
**characterized in that**
- the laboratory sample distribution system (100) comprises underfloor storage means (60) to temporarily store a number of sample container carriers (140) or sample containers (145), wherein the underfloor storage means (60) are arranged inside the underfloor chamber (30).

12. Laboratory (10) according to one of the preceding claims,
**characterized in that**
- the drive means (120) are embodied as electro-magnetic actuators (120), and
- each sample container carrier (140) comprises a magnetically active device (141).

13. Laboratory (10) according to one of the preceding claims,
**characterized in that**
- the transport plane (110) is arranged solely inside the underfloor chamber (30),
- at least one of the stations (106) is arranged in the underfloor chamber (30), and
- at least one of the stations (105) is arranged above the underfloor chamber (30).
